# EUROPEAN PATENT APPLICATION

(11) **EP 1 099 754 A1**
(43) Date of publication of application: **16.05.2001**
(21) Application number: 99203761.4
(22) Date of filing: 10.11.1999
(51) Int. Cl.: C12N 5/06, C12N 5/08, C12N 5/10, A61K 48/00, A61K 35/14, A61K 35/28, A61K 35/42, A61P 43/00

(54) **Mesenchymal stem cells and/or progenitor cells, their isolation and use**

(71) Applicant: Universiteit Leiden, 2312 AV Leiden (NL)
(72) Inventor: Falkenburg, Johan Herman Frederik, 2341 ET Oestgeest (NL)
(74) Representative: Ottevangers, Sietse Ulbe

(57) **Abstract**

An isolated mesenchymal stem cell and/or progenitor cell obtainable from fetal lung tissue and/or from fetal bone marrow and or/from umbilical cord blood, further characterized by being CD34+, CD45-, THY-1+ and AS-02+, uses thereof; a method for partial reconstitution of a group of mesenchymal cells in a patient, that could comprise a therapeutic gene.

## Description

The present invention relates to the field of medicine, in particular to transplantation or reconstitution of certain tissues and/or cells, optionally in combination with gene therapy.

Reconstitution of tissues can be obtained by transplanting complete organs like kidneys or lungs or liver, or by transplanting groups of cells which can grow out in vivo to form tissues, a concept well known for hematopoietic stem cells. In fact hematopoietic stem cells are defined by their capability of long term repopulation (reconstitution) of the hematopoietic system of a host. In order to be able to reconstitute stem cells need to be pluripotent (omnipotent) and capable of proliferation into all potencies required. There is another group of pluripotent hematopoietic cells, which cells are called progenitor cells. The distinction is relatively arbitrary. Where pluripotent stem cells are supposed to be able to proliferate and differentiate into many different cell lineages, progenitor cells are often stated to be capable of differentiating into a limited number of lineages. The distinction as said, is however not clear. When a stem cell (after how many divisions, which signal?) becomes a progenitor cell is an academic question. For the purpose of this invention both stem cells and progenitor cells are intended where either is mentioned, unless clearly indicated otherwise. As stated herein before the concept of reconstituting certain tissues or groups of cells is well known for hematopoietic lineages. However, this concept can be extended but is not known outside this field for lack of pluripotent stem cells of other than hematopoietic origin. Mesenchymal stem cells have been defined by their ability to give rise to multiple cell lineages including fibroblast, bone, adipocytes, and cartilage. These cells have been shown to be present in adult bone marrow. However, the phenotype of these mesenchymal stem cells is not clearly defined.

The present inventors have identified, isolated and characterised a stem cell for mesenchymal cells and/or tissues.

Thus the present invention provides an isolated mesenchymal stem cell and/or progenitor cell obtainable from fetal lung tissue and/or from bone marrow and/or fetal blood, including umbilical cord blood. Other sources may become available as well, now that their characteristics (as described herein) have been given.

According to the invention a good source for these mesenchymal stem and/or progenitor cells is provided in fetal lung tissue. Cells expected to be mesenchymal stem cells can thus be compared to stem cells isolated and characterised as mesenchymal stem cells from fetal lung tissue. The mesenchymal stem cells according to the invention preferably share a characteristic, which can be used for their identification, with hematopoietic stem cells.

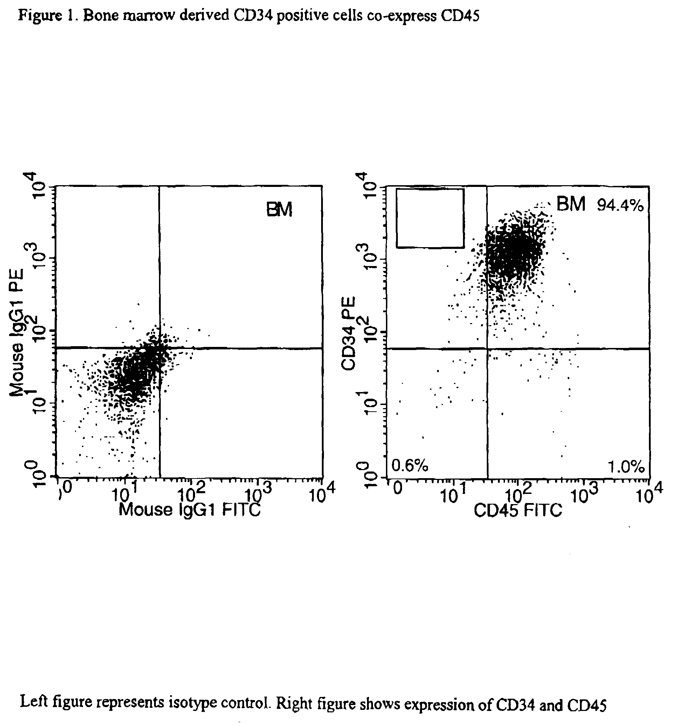

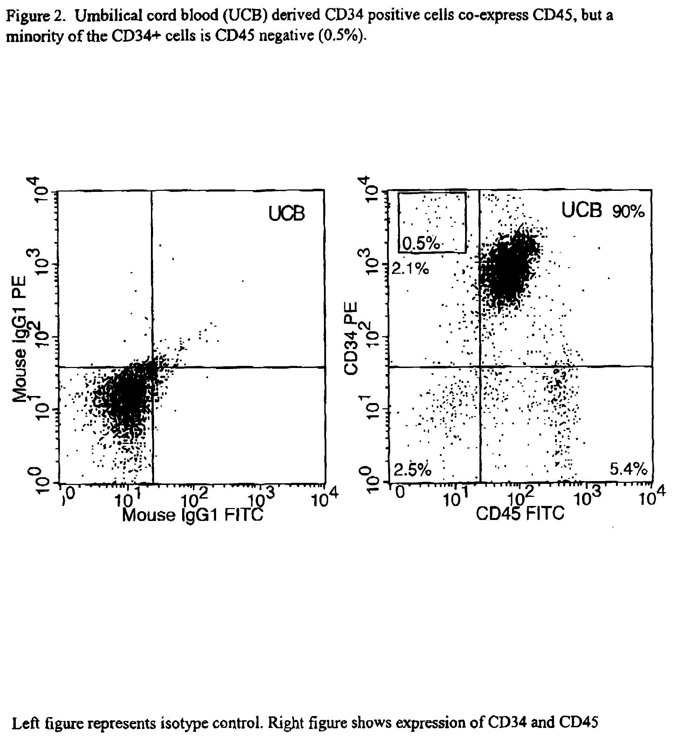

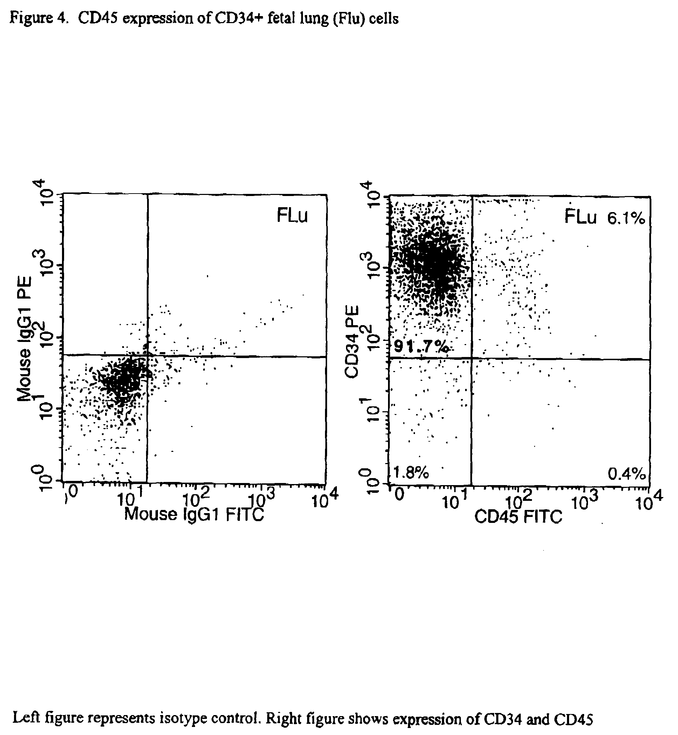

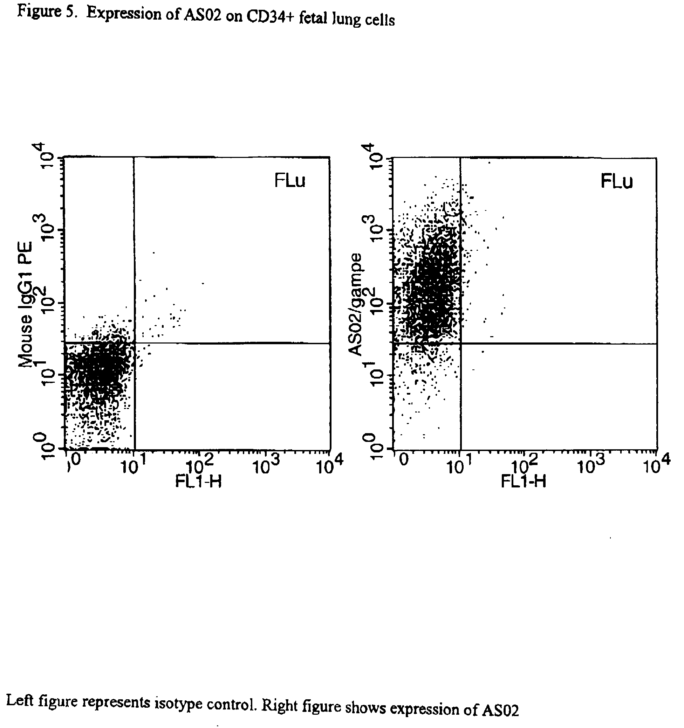

Hematopoietic stem cells defined as precursor cells capable of giving rise to a progeny of mature hematopoietic cells of different lineages are amongst others characterized by certain membrane markers. CD34 has been shown to be present on a majority of hematopoietic stem cells. The frequencies of CD34+ cells in grafts that are used for clinical hematopoietic stemcell transplantation are shown in table 1. The mesenchymal stem cells according to the invention in general also have this CD34 membrane marker.Cells from hematopoietic origin however, further express the CD45 antigen on their cell membrane. Indeed, as shown in figure 1, all CD34+ cells in adult bone marrow co-express CD45. In contrast, in umbilical cord blood (UCB) and in fetal bone marrow a small minority of the CD34+cells does not express CD45 (Figure 2 and 5). The origin of these CD34+CD45- cells, which did not proliferate in vitro in response to hematopoietic growth factors is unknown. Since the exact phenotype of hematopoietic stem cells during ontogeny is largely unknown, attempts were made to characterize the CD34+ stem cell content of several fetal tissues. In fetal lung tissue a high frequency of a group of CD34 positive cells was found which was negative for the CD45 marker (figure 4). These cells have been shown in the present invention to be mesenchymal stem cells. Thus the invention provides in a preferred embodiment an isolated mesenchymal stem cell and/or progenitor cell characterised by expression of the membrane marker CD34 and by the absence of natural expression of CD45. Together with ( a comparison with stem cells from) the source these two markers should suffice to identify mesenchymal stem and/or progenitor cells according to the invention. These cells are in general capable of expansion (or proliferation or outgrowth) in many (if not all) of the mesenchymal lineages. Important uses of these cells according to the invention are thereby immediately apparent. Cartilage and bone for instance are among these lineages. Outgrowth into (artificial) bone and/or (artificial) cartilage for replacement of damaged or disappeared tissue is thus a first apparent use. In vivo reconstitution of mesenchymal tissues can also be envisaged. Furthermore gene therapy can also be accomplished by providing cells according to the invention with a gene of interest. Mesenchymal stem cells and/or progenitor cells are thus further defined by their capability to at least reconstitute a part of missing or defective mesenchymal lineages in a host.

Further identification of a number of cells according to the invention is provided by two further markers which can distinguish the cells according to the invention from e.g. endothelial cells. Thus the invention provides in a further embodiment a cell according to the invention, which carries a marker identified as THY-1, as well as a cell according the invention, which carries a marker identified as AS-02. Apart from the isolated cells which may of course be isolated by using their markers, the invention also provides therapeutic methods using these cells. Thus the invention provides a method for at least partial reconstitution of a group of mesenchymal cells in a patient, comprising providing a cell according to the invention, matching said cell with the patient according to HLA-type, optionally allowing for clonal outgrowth and some differentiation of outgrown cells and administering said cell or cells to said patient. Clonal outgrowth, expansion and/or proliferation may reduce the pluripotency of the invidual stem cells, but the pluripotency will remain present in the progeny as a whole over a number of generations. Even loss of pluripotency may not be a problem and some differentiation in certain directions (using signals) may even be advantageous (e.g. for bone formation and the like). A preferred application of the cells according to the invention is in bone formation. Thus the invention provides a method according to the invention, wherein said group of mesenchymal cells to be reconstituted comprises bone cells such as osteoblasts. Other methods according to the invention are applied for the reconstitution (if only partial) of adipocytes, and/or fibroblasts and/or muscle cells. According to the invention the stem cells can also be used in gene therapy regimes. Thus the invention also provides a method according to the invention, wherein said stem cell according to the invention is provided with a gene of interest. Methods for delivering genes are well known in the art and need hardly be explained here. However in terms of long term repopulation typically one would employ long term gene expression of the gene of interest, thereby aiming at corredction of e.g. deficiencies. For long term expression integration of the gene of interest in the genome of the host is preferred. This can be achieved through certain viral vectors such as those based on retroviruses ar adeno associated viruses. Such vectors and other means of gene delivery are often referred to as gene delivery vehicles. Genes of interest may be therapeutic genes, but also genes encoding cytostatic substances or even genes leading to antisense RNA, etc.

A major advantage of gene therapy using stem cells according to the invention is that it may be carried out ex vivo. A stem cell can be isolated from the body, provided with the gene of interest (then expanded) and administered to the patient. The altered cell is also part of the present invention. Thus the invention provides a cell obtainable by a method according to the invention.

The use of these cells in therapy is also provided by the present invention. Be it in gene therapy or as partial reconstitution in a kind of transplantation like manner. Thus the invention also provides the use of a cell according to the invention in the manufacture of a medicament for at least partial reconstitution of a group of mesenchymal cells in a patient, in particular wherein said group of mesenchymal cells are adipocytes, fibroblasts, muscle cells and/or bone cells.

Also included are methods to increase the number of stem cells and/or progenitor cells in order to increase chances of long term repopulation. Thus the invention also provides a method for providing a plurality of mesenchymal stem and/or progenitor cells suitable for at least partial reconstitution of a group of mesenchymal cells in a patient, comprising providing a mesenchymal stem and/or progenitor cell according the invention, culturing said cell in a suitable medium, allowing for proliferation of said cell and harvesting the resulting plurality of mesenchymal stem and/or progenitor cells. Included are also such methods wherein said cell or its progeny is provided with a differentiation signal. Also provided is a plurality of CD34+, CD45-, mesenchymal stem and/or progenitor cells obtainable by a method according to the invention. The invention will be explained in more detail in the following experimental part.

### Experiments.

### Introduction:

Hematopoietic stem cells defined as precursor cells capable of giving rise to a progeny of mature hematopoietic cells of different lineages are amongst others characterized by certain membrane markers. CD34 has been shown to be present on a majority of hematopoietic stem cells. The frequencies of CD34+ cells in grafts that are used for clinical hematopoietic stemcell transplantation are shown in table 1. Cells from hematopoietic origin express the CD45 antigen on their cell membrane. Indeed, as shown in figure 1, all CD34+ cells in adult bone marrow co-express CD45. In contrast, in umbilical cord blood (UCB), a small minority of the CD34+cells does not express CD45 (Figure 2). The origin of these CD34+CD45- cells, which did not proliferate in vitro in response to hematopoietic growth factors is unknown. Since the exact phenotype of hematopoietic stem cells during ontogeny is largely unknown, attempts were made to characterize the hematopoietic stem cell content of several fetal tissues.

### CD34 positive cells in fetal tissues:

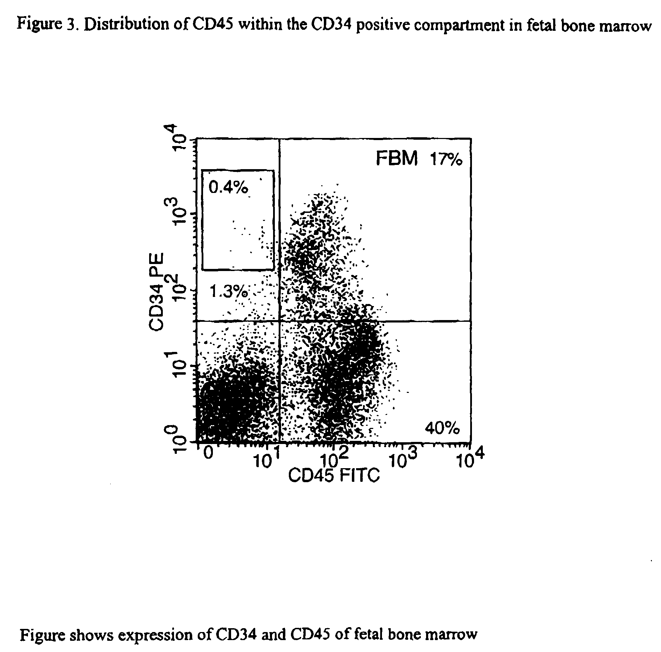

Fetal hematopoietic tissues contain high frequencies of CD34+ cells (Table 2). A relatively high frequency of CD34+ cells co-expressing the hematopoietic specific lineage marker CD45 was shown to be present in fetal bone marrow, fetal liver, spleen, blood and thymus (Figure 3, Table 3). In addition to the presence in these classical hematopoietic tissues, a high frequency of CD34+ cells was found in fetal lung tissue (Table 2). In contrast to the majority of CD34+ cells in the hematopoietic tissues, fetal lung derived CD34+ cells were shown to be largely CD45- (Figure 4, Table 3). Only a small minority of the CD34+ cells was CD45+, which may in part be due to the presence of blood-derived hematopoietic precursor cells. These results indicated that fetal lung may contain at relatively large frequencies CD34+ precursor cells not belonging to the hematopoietic lineages. Therefore, attempts were made to characterize these fetal lung derived CD34+ precursor cells.

### CD34+CD45- fetal lung cells:

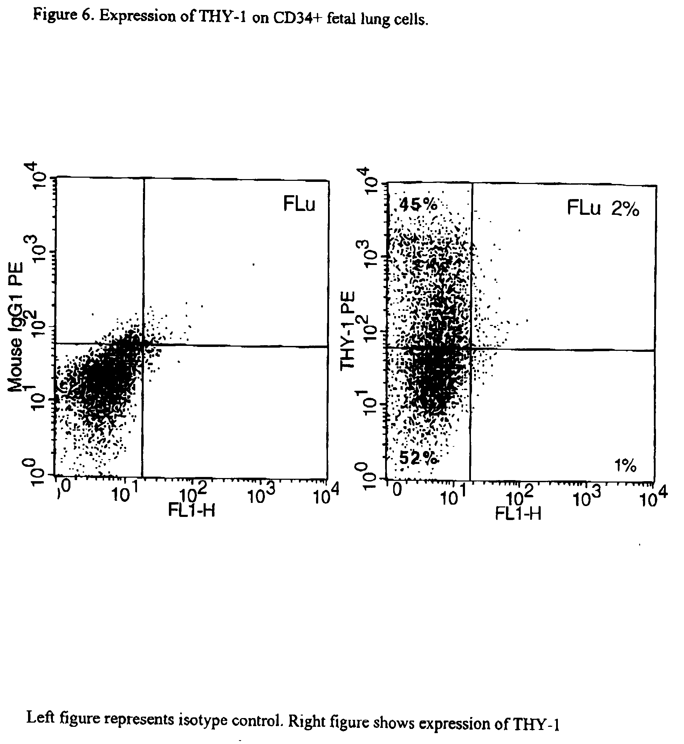

Whereas hematopoietic stem cells may be capable of correcting disorders of the hematopoietic system, mesenchymal stem cells may give rise to cells of mesenchymal lineages including bone, cartilage, muscle and fat tissue. Analogous to hematopoietic stem cells, mesenchymal stem and progenitor cells may be used to possibly correct disorders of bone, cartilage or muscle. Identification of mesenchymal stem cells may lead to expansion of those cells in vitro, and may be used to generate cells of various mesenchymal lineages for clinical use. We hypothesized that the CD34+ progenitor cells derived from fetal lung tissues were mesenchymal stem cells capable of proliferation, and differentiation into multiple mesenchymal lineages. As illustrated in table 2, the frequency of CD34+ cells in fetal lung tissue is high. The majority of these cells were shown to lack the hematopoiesis specific lineage marker CD45. Further characterization of this CD34+CD45- cell revealed that the majority of these cells were positive for the markers THY-1 and ASO-2 (Figures 5 and 6). AS-02 has been shown to be restricted to cells leading to proliferation of cells resembling fibroblasts, and were not expressed on CD34+ endothelial cells illustrating that the CD34+CD45- cells from fetal lung were not endothelial cells. The CD34+CD45- cells were shown to be only weakly expressing HLA-1 molecules.

### Functional analysis:

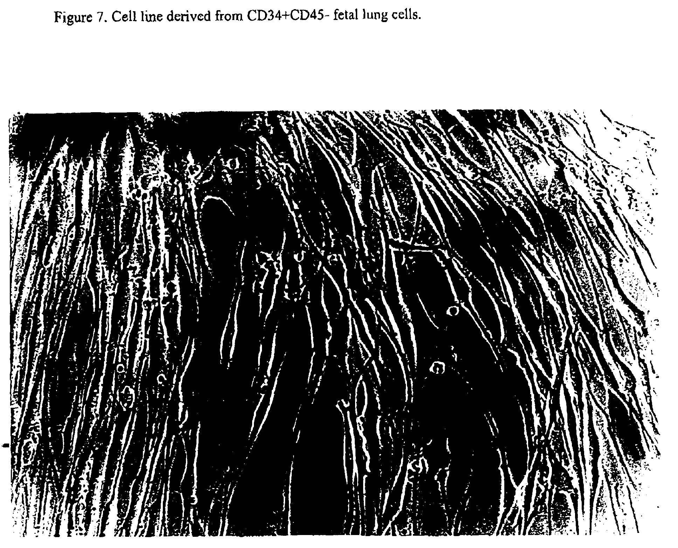

After purification of the CD34+ cells from fetal lung tissue to exclude contamination of CD34- cells, the cells were plated in tissue cultured micro titer plates coated with gelatine and stimulated with M199 standard medium containing 10% fetal calf serum (FCS) in the presence of endothelial cell growth factor (ECGF). After one week of culture these cells were rapidly proliferating into cell lines resembling fibroblast like cells were established (Figure 7). These cells were shown to express AS-02, again illustrating that these cells were not endothelial cells. Rapidly proliferating cell lines could be obtained not only from fetal lung tissue, but also bone marrow derived accessory cell lines could be established, whereas only limited proliferation of similar cells was found in cultures of fetal liver or spleen cells (Table 4).

### Multi lineage capacity of CD34+ mesenchymal stem cells from fetal lung tissue:

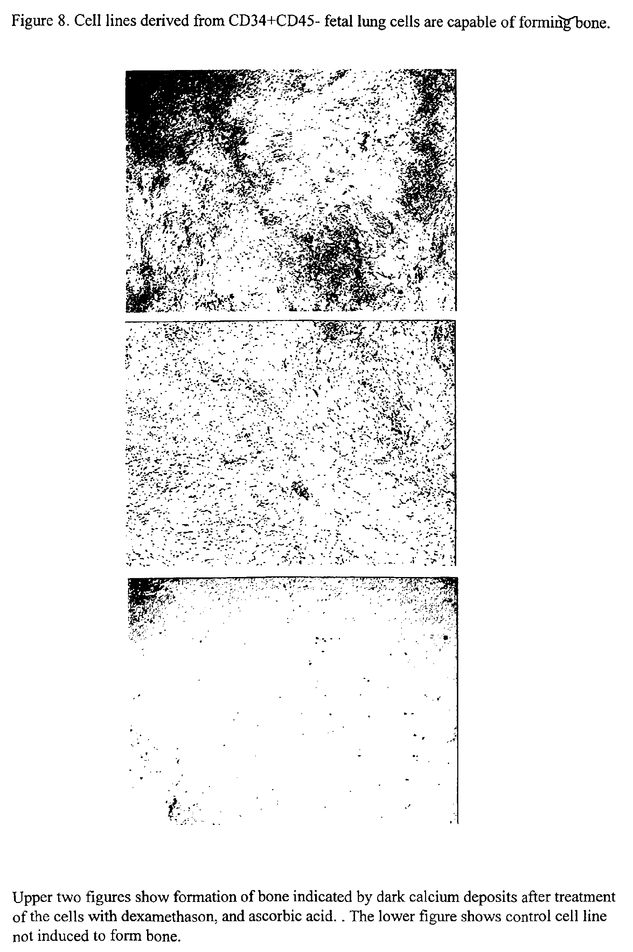

To investigate the multi-lineage capacity of the CD34+ mesenchymal stem cells derived from fetal lung tissue, these cells were cultured in the presence or absence of 10⁻⁷ M dexamethasone and 50 ug/ml ascorbic acid, and from day 7 of culture 5mM b-glycerophosphate. As illustrated in Figure 8, the cells could be induced to form bone. In addition, these mesenchymal cells could be induced to differentiate into adipocytes. These results illustrate the multi potential capacity of the fetal lung derived CD34+CD45- cells.

**Table 1**

| . Percentages of CD34+ cells in several tissues as determined according to the SIHON-method. | | |
|---|---|---|
| Source | % of CD34⁺ cells | |
| UCB | 0.28 ± 0.26 | (n=9) |
| BM | 1.12 ± 0.54 | (n=6) |
| mPB | 1.06 ± 0.7 | (n=9) |

**Table 2**

| . Percentages of CD34⁺ cells in several fetal tissues as determined according to the SIHON-method. | |
|---|---|
| Source | % of CD34⁺ cells |
| Fetal lung | 36 ± 19 (n=8) |
| Fetal BM | 22 ± 5.5 (n=10) |
| Fetal liver | 5.0 ± 2.8 (n=11) |
| Fetal spleen | 8.4 ± 6.3 (n=11) |

**Table 3**

| . Percentages of CD34⁺CD45⁻ cells within isolated CD34+ cells from several sources | |
|---|---|
| Source | % CD34⁺CD45⁻ of isolated CD34⁺ cells |
| Fetal lung | 90 ± 3.8 (n=6) |
| Fetal BM | 2 ± 2 (n=2) |
| UCB | 0.47 ± 0.36 (n=15) |
| BM | 0 (n=6) |
| mPB | 0 (n=10) |

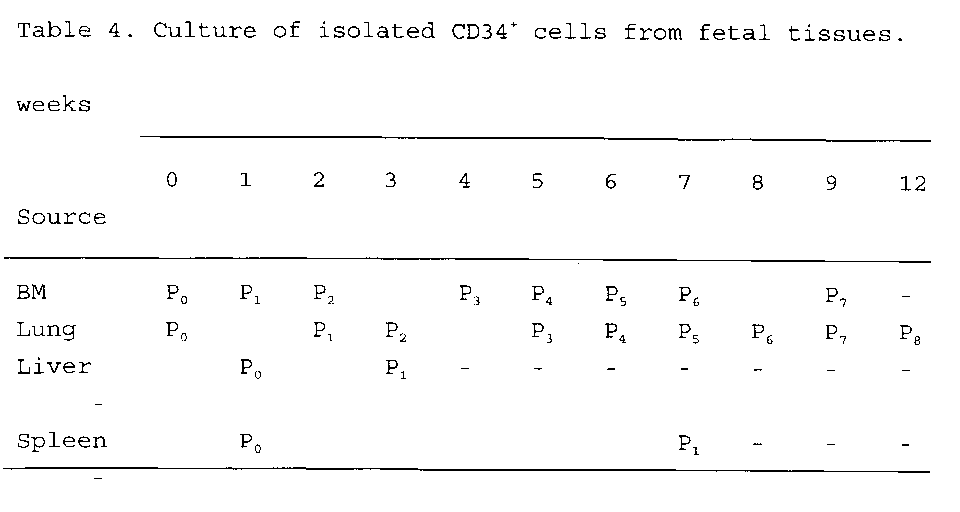

When cells grew to confluency they were trypsinized and replated. P indicates the number of passages that were obtained.

### Legends to the figures

Figure 1. Bone marrow derived CD34 positive cells co-express CD45

Left figure represents isotype control. Right figure shows expression of CD34 and CD45

Figure 2. Umbilical cord blood (UCB) derived CD34 positive cells co-express CD45, but a minority of the CD34⁺ cells is CD45 negative (0.5%).

Figure 3. Distribution of CD45 within the CD34 positive compartment in fetal bone marrow
Figure shows expression of CD34 and CD45 of fetal bone marrow

Figure 4. CD45 expression of CD34⁺ fetal lung (Flu) cells

Left figure represents isotype control. Right figure shows expression of CD34 and CD45

Figure 5. Expression of AS02 on CD34⁺ fetal lung cells

Left figure represents isotype control. Right figure shows expression of AS02

Figure 6. Expression of THY-1 on CD34⁺ fetal lung cells.

Left figure represents isotype control. Right figure shows expression of THY-1

Figure 7. Cell line derived from CD34+CD45- fetal lung cells.

Figure 8. Cell lines derived from CD34+CD45- fetal lung cells are capable of forming bone

Upper two figures show formation of bone indicated by dark calcium deposits after treatment of the cells with dexamethason, and ascorbic acid. . The lower figure shows control cell line not induced to form bone.

### REFERENCES

1. Caplan A.I., The mesengenic process. Clinics in plastic surgery 11: 429-435, 1994
2. Waller E.K., Olweus J., Lund-Johansen, F., Huang S., Nguyen M., Guo G., Terstappen L., The common stem cell hypothesis reevaluated: Human fetal bone marrow contains separate populations of hematopoietic and stromal progenitors. Blood 85: 2422-2435, 1995
3. Thomson J.A., Itskovitz-Eldor J., Shapiro S.S., Waknitz M.A., Swiergiel J.J., Marshall V.S., Jones J.M., Embryonic stem cell lines derived from human blastocysts. Science 282: 1145-1147, 1998
4. Pittenger M.F., Mackay A.M., Beck S.C., Jaiswal R.K., Douglas R., Mosca J.D., Moormann M.A., Simonetti D.W., Craig S., Marshak D.R., Multilineage potential of adult human mesenchymal stem cells. Science 284: 143-147, 1999
5. Horwiz E.M., Prockop D.J., Fitzpatrick L.A., Koo W.W.K., Gordon P.L., Neel M., Sussman M., Orchard P., Marx J.C., Pyeritz R.E., Brenner M.K. Transplantability and therapeutic effects of bone marrow-derived mesenchymal cells in children with osteogenesis imperfecta. Nature Medicine Vol. 5, no. 3: 309-313, 1999

## Claims

1. An isolated mesenchymal stem cell and/or progenitor cell obtainable from fetal lung tissue and/or from fetal bone marrow and/or from umbilical cord blood.

2. An isolated mesenchymal stem cell and/or progenitor cell according to claim 1, characterised by expression of the membrane marker CD34.

3. An isolated mesenchymal stem and/or progenitor cell according to claim 1 or 2, characterised by the absence of natural expression of CD45.

4. A cell according to any one of claims 1-3, which carries a marker identified as THY-1.

5. A cell according to any one of claims 1-4, which carries a marker identified as AS-02.

6. A method for at least partial reconstitution of a group of mesenchymal cells in a patient, comprising providing a cell according to any one of claims 1-5, optionally matching said cell with the patient according to HLA-type, optionally allowing for clonal outgrowth and differentiation of outgrown cells and administering said cell or cells to said patient.

7. A method according to claim 6, wherein said group of mesenchymal cells comprises bone cells such as osteoblasts.

8. A method according to claim 6, wherein said group of mesenchymal cells comprises adipocytes.

9. A method according to claim 6, wherein said group of mesenchymal cells comprises fibroblasts, muscle cells and/or cartilage.

10. A method according to any one of claims 6-9, wherein said cell according to claims 1-5 is provided with a gene of interest.

11. A method according to claim 10, wherein said gene of interest is a therapeutic gene.

12. A method according to claim 10 or 11, wherein said gene is delivered through a gene delivery vehicle.

13. A cell obtainable by a method according to any one of claims 6-12.

14. A cell according to any one of claim 1-5 or 13 for use as a medicament.

15. Use of a cell according to any one of claims 1-5 or 13 in the manufacture of a medicament for at least partial reconstitution of a group of mesenchymal cells in a patient.

16. Use according to claim 15, wherein said group of mesenchymal cells are adipocytes, fibroblasts and/or bone cells, muscle cells and/or cartilage.

17. A method for providing a plurality of mesenchymal stem and/or progenitor cells suitable for at least partial reconstitution of a group of mesenchymal cells in a patient, comprising providing a cell according to any one of claims 15 or 13, culturing said cell in a suitable medium, allowing for proliferation of said cell and harvesting the resulting plurality of mesenchymal stem and/or progenitor cells.

18. A method according to claim 17 further comprising a step wherein said cell or its progeny is provided with a differentiation signal.

19. A plurality of CD34+, CD45-, mesenchymal stem and/or progenitor cells obtainable by a method according to any one of claims 17 or 18.
